(19) Europäisches Patentamt
European Patent Office
Office européen des brevets

(11) Veröffentlichungsnummer: **0 268 039 B1**

(12) # EUROPÄISCHE PATENTSCHRIFT

(45) Veröffentlichungstag der Patentschrift: **02.09.92**

(51) Int. Cl.5: **C07C 69/732**, C07C 69/84, C07C 323/31, C08K 5/13, C07C 323/10

(21) Anmeldenummer: **87113762.6**

(22) Anmeldetag: **21.09.87**

(54) **Ester von 3-tert.Butyl- bzw. 3-tert.-Butyl-5-alkyl-4-hydroxyphenyl-(alkan-)-carbonsäuren mit Oxethylaten von Polythiolen, Verfahren zu ihrer Herstellung und ihre Verwendung als Stabilisatoren.**

(30) Priorität: **18.11.86 DE 3639353**

(43) Veröffentlichungstag der Anmeldung:
**25.05.88 Patentblatt 88/21**

(45) Bekanntmachung des Hinweises auf die Patenterteilung:
**02.09.92 Patentblatt 92/36**

(84) Benannte Vertragsstaaten:
**BE CH DE FR GB IT LI NL**

(56) Entgegenhaltungen:
**EP-A- 0 004 762**
**EP-A- 0 102 920**
**EP-A- 0 137 395**
**EP-A- 0 165 209**
**US-A- 3 896 146**

(73) Patentinhaber: **HÜLS AKTIENGESELLSCHAFT**
**Patentabteilung / PB 15 - Postfach 13 20**
**W-4370 Marl 1(DE)**

(72) Erfinder: **Küpper, Friedrich-Wilhelm, Dr.**
**Oderbruchstrasse 27**
**W-4370 Marl(DE)**
Erfinder: **Voges, Hans-Werner, Dr.**
**Im Gorden 45**
**W-4270 Dorsten 21(DE)**
Erfinder: **Haage, Hans-Jürgen, Dr.**
**Gaussstrasse 13**
**W-4690 Herne 2(DE)**

EP 0 268 039 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind neue, nicht vorbeschriebene Ester von 3-tert.Butyl- bzw. 3-tert.Butyl-5-alkyl-4-hydroxyphenyl-(alkan-)carbonsäuren der Formel I

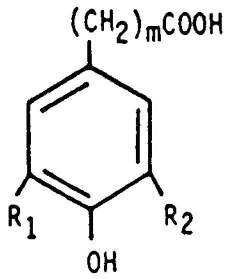

(I)

mit

$R_1$ = $-C(CH_3)_3$,

$R_2$ = H,$C_1$- bis $C_4$-Alkyl, vorzugsweise $-C(CH_3)_3$,

m = 0 bis 4, vorzugsweise 2,

mit Oxethylaten von Polythiolaromaten der allgemeinen Formeln II und III, vorzugsweise der Formel II, mit 2 bis 6 Thiolgruppen

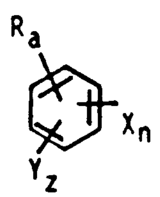

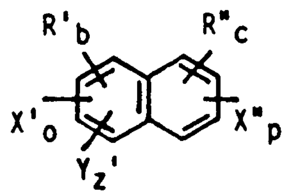

(II) (III)

sowie mit Oxethylaten von Dithiolcycloalkanen der allgemeinen Formel IV, die maximal 6 Alkylenoxideinheiten enthalten,

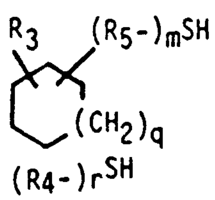

(IV)

mit $X = (CH_2)_m SH$, $X' = (CH_2)_r SH$, $X'' = (CH_2)_s SH$ wobei m,r,s = 0 oder 1 sein können

R,R',R" = H, Alkyl, Halogen, Hydroxyl,
$R_3$ = H, $C_1$- bis $C_4$-Alkyl,
$R_4$,$R_5$ = $C_2$- bis $C_4$-Alkylen,
q = 0 bis 7, vorzugsweise 1,

Y = 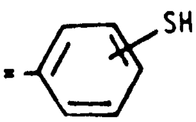

n = 2 bis 6 für z bzw. z' = 0,
n = 1 bis 5 für z bzw. z' ≠ 0,
o + p = n
wobei
$0 \leqq a, z \leqq a + z \leqq 6\text{-}n$ (in Formel II)
$0 \leqq b, c, z' \leqq 4 \leqq 8\text{-}n$ (in Formel III)
sein können und die Anwesenheit einer einzelnen OH-Gruppe in Formel II ausgeschlossen ist und die Verwendung derartiger Ester zur Stabilisierung von organischen Polymeren, vorzugsweise von Polyolefinen.

Es ist bekannt, daß organische Polymere, wie sie durch Polymerisation (bzw. Copolymerisation) von ggf. funktionelle Gruppen enthaltenden Mono- und Diolefinen oder durch Polykondensation geeigneter Vorstufen - beispielsweise von Diolen mit Dicarbonsäuren - erhalten werden, unter der Einwirkung von Luft/Sauerstoff, von Wärme, von Licht oder von energiereicher Strahlung Veränderungen erleiden können, die die anwendungstechnisch wichtigen Eigenschaften der Polymeren wie Festigkeit, Härte und Dehnung beeinträchtigen. Durch derartige Schädigungen tritt häufig nicht nur eine deutliche Veränderung der meßbaren physikalischen Eigenschaften ein, sondern es kommt zu einer auch visuell bemerkbaren Erweichung, Versprödung und/oder Verfärbung der Fertigteile. Aus diesem Grunde werden den entsprechenden Polymeren vor der Verarbeitung Stabilisatoren zugesetzt. Hinsichtlich näherer Einzelheiten verweisen wir auf die zusammenfassenden Arbeiten von G. Scott, "Atmospheric Oxidation and Antioxidants", Elsevier Publ. Co.; Amsterdam, Oxford, New York (1965); R. Gächter, H. Müller, "Taschenbuch der Kunststoff-Additive", C. Hanser-Verlag, München/Wien (1979), J. Pospisil in "Degradation and Stabilization of Polymers", (Edit.: H. Jellinek), Elsevier, Amsterdam, Oxford, New York (1983), S. 193 ff.; P. P. Klemchuk et al., "Polymer Degradation and Stabilization" 7 (1984), S. 131 ff.

Es ist auch bekannt, daß in Polyolefinen als Stabilisatoren vorzugsweise phenolische Hydroxylgruppen enthaltende Verbindungen verwandt werden und daß unter diesen Phenolderivaten Substanzen mit voluminösen Alkylgruppen, vorzugsweise mit tert. Butylsubstituenten, in mindestens einer ortho-Stellung zur phenolischen Hydroxylgruppe eine besonders gute Wirksamkeit aufweisen. Unter der Vielzahl der beschriebenen Stabilisatoren (vgl. z. B. J. C. Johnson, "Antioxidants", Noyes Data Corp., 1975; M. W. Ramsey, "Antioxidants-Recent Developments", Noyes Data Corp., 1979; M. T. Gillies, "Stabilizers for Synthetic Resins", Noyes Data Corp., Park Ridge, N. J., 1983) sind auch Ester zu finden. Ester von 3,5-Dialkyl-4-hydroxy-phenyl-(alkan-)carbonsäuren werden beispielsweise in den US-PSS 3 681 431, 3 330 859; 3 644 482, 3 285 855, Ester der 4,6-Dialkyl-3-hydroxyphenyl-(alkan-)carbonsäuren in den US-PSS 3 988 363, 3 862 130 und Ester der 2-Methyl-4-tert.-butyl-5-hydroxyphenylalkan-carbonsäuren in der EP-PS 0 048 841 beschrieben.

Des weiteren beschreibt die EP-A 165 209 eine Vielzahl von o,p-bifunktionalisierten, o'-substituierten Phenolen als Stabilisator. Es handelt sich stets um Monophenole. Unter anderem wird ein 6-tert.-Butylphenol gezeigt, das in o- und p-Stellung mit 3,5-Di-tert.-butyl-4-hydroxyphenyl-propionsäure veresterte (2-Hydroxyethyl)-thiomethylgruppen enthält.

Die Wirkung der phenolischen Stabilisatoren läßt sich durch Zusatz bestimmter, meist schwefel- oder phosphorhaltiger Verbindungen häufig noch weiter steigern. Das optimale Mengenverhältnis von Stabilisator und Synergist ist im Einzelfall stets experimentell zu ermitteln. Sowohl für die Stabilisatoren als auch für die

Synergisten gilt, daß sie sich ohne Schwierigkeiten unzersetzt in die verschiedenen Polymeren einarbeiten und darin möglichst gleichmäßig verteilen lassen sollten. Andererseits sollte sich das Polymer bei den erforderlichen hohen Einarbeitungstemperaturen weder durch Zusätze verfärben noch durch die Temperatur- und Scherbeanspruchung einen Molekulargewichtsabbau erleiden.

Aus der Literatur sind verschiedene Verbindungen bekannt, die als Stabilisatoren eingesetzt werden und die neben sterisch abgeschirmten Hydroxyphenylgruppen synergistisch wirkende Schwefelatome im gleichen Molekül enthalten, (vgl. z. B. R. W. Layer, "Non-staining Antioxidants" (in G. Scott, "Developments in Polymer Stabilization" 4 (1981), S. 163 ff., S. 167); F. X. O'Shea in "Advances Chem. Series" 85 (1968), S. 128 ff.; G. Scott in "Developments in Polym. Stabilization" 6 (1983), S. 29 ff.). Vor allem Abkömmlinge des ortho-Thiobisphenols sollen zu starker Verfärbung neigen und werden wohl nur zur Stabilisierung von Kautschukmischungen verwandt.

Die Aufgabe der Erfindung bestand darin, ausgehend von an sich bekannten 3-tert.Butyl- bzw. 3-tert.Butyl-5-alkyl-4-hydroxyphenyl-(alkan-)carbonsäuren oder deren Derivaten, vorzugsweise von 3-(3,5-Di-tert.butyl-4-hydroxyphenyl-)propionsäure(derivate)n, durch Umsetzung mit ausgewählten, wirtschaftlich herzustellenden und Schwefel als Heteroatom enthaltenden Alkoholen zu Estern dieser 3-tert.Butyl- bzw. 3-tert.Butyl-5-alkyl-4-hydroxyphenyl-(alkan-)carbonsäuren der Formel I mit verbesserten Stabilisatoreigenschaften zu gelangen und durch Einarbeitung dieser Ester in organische Polymere, vorzugsweise in Polypropylen und Polyethylen, eine gute Stabilisierung derartiger Polymerer gegen Molekulargewichtsabbau bei der Verarbeitung oder beim Einsatz über längere Zeit zu erreichen, ohne daß eine nennenswerte Verfärbung als Folge des Stabilisatorzusatzes bei diesen Beanspruchungen eintritt.

Die Lösung dieser Aufgabe gelingt überraschend, wenn die leicht zugänglichen Oxethylate von Polythiolaromaten mit 2 bis 6 Thiolgruppen der allgemeinen Formeln II und III, vorzugsweise der Formel II, mit 2 bis 4 Thiolgruppen oder von Dithiolcycloaliphaten der Formel IV in die Ester von (3-tert.Butyl- bzw. 3-tert.Butyl-5-alkyl-)-4-hydroxyphenyl-(alkan-)carbonsäuren überführt werden und diese, vorzugsweise die Oxethylate mit einer Alkylenoxideinheit pro Thiolgruppe als Alkoholkomponente enthaltenden Ester zur Stabilisierung organischer Polymerer, vorzugsweise von Polyolefinen, eingesetzt werden.

Bevorzugt verwandt werden Ester, in denen sämtliche Thiolgruppen der Polythiolaromaten bzw. Dithiolcycloaliphaten mit je einem Alkylenoxid, vorzugsweise Ethylenoxid, oxethyliert wurden und in denen sämtliche der dabei entstandenen 2-Hydroxy-(2-alkyl-)ethyl-thioethergruppen verestert sind. Ganz besonders bevorzugt werden Ester der 3-(3,5-Di-tert.butyl-4-hydroxyphenyl-)propionsäure mit Poly-(monooxethylaten), d. h. Poly-(2-hydroxyethylthioethern), von Polythiolaromaten der Formel II.

Diese Lösung der Aufgabe ist außerordentlich überraschend, da aus der Literatur bekannt ist, daß die C-S-Bindung in Thioethern eine geringere Bindungsstärke als C-C-Bindungen besitzt. (vgl. z. B. W. Tagaki, "Sulfides" in S. Oae, "Organic Chemistry of Sulfur" (1977) S. 245 ff.). Es war daher nicht vorherzusehen, daß mit den erfindungsgemäßen Estern auf der Grundlage von Polythiolaromaten der Formel II und III bzw. von Cycloalkandithiolen der Formel IV Stabilisatoren erhalten werden können, die mindestens zwei derartige Thioetherbindungen enthalten und die sich dennoch bei den hohen Temperaturen der Polyolefin-Verarbeitung unzersetzt einarbeiten lassen, wobei neben guter Wirksamkeit der Stabilisatoren nur eine überraschend geringe Verfärbung der damit versehenen Polymermaterialien zu beobachten ist.

Die Eigenschaften der erfindungsgemäßen Stabilisatoren, die Aryl- oder Cycloalkylsubstituenten in der schwefelhaltigen Polyolkomponente enthalten, überraschen umso mehr, da nach dem Stand der Technik (vgl. US-PS 3 441 575, BE-PS 637 444) herstellbare, z. T. im Handel erhältliche Ester von 3,5-Dialkyl-4-hydroxyphenyl-(alkan-)carbonsäuren mit monofunktionellen Alkylthioalkoholen oder Thioalkandiolen der Formeln

$RS(CH_2)_nOH$ bzw. $HO(CH_2)_mS(CH_2)_nOH$ (mit m, n > 1)

in Polyolefinen zu erheblicher Verfärbung neigen, wie ein Vergleichsbeispiel (s. Beispiel 37) belegt. Andererseits handelt es sich vor allem bei den Abkömmlingen der stets nur ein Schwefelatom enthaltenden Alkohole mit relativ kurzer aliphatischer Kette ($R < C_8$) überwiegend um Flüssigkeiten. Diese sind zur Einarbeitung in Polyolefine i. a. nicht gut geeignet, da die gleichmäßige Verteilung und der kontinuierliche Zusatz der notwendigen kleinen Mengen Schwierigkeiten bereiten.

Erstaunlich ist die Wirkung der erfindungsgemäßen Ester als Stabilisatoren von vorzugsweise Polyolefinen auch deshalb, weil nach dem Stand der Technik Polythiolaromaten der Formel II mit m = 0 und z = 0 z. T. außerordentlich starke Reduktionsmittel sind, die mit Sauerstoff umgehend reagieren und die daher schwierig, am besten unter anaeroben Bedingungen, zu handhaben sind (vgl. z. B. F. Wudl et al., J. Org. Chem. 50 (1985), 2395). Es war daher nicht ohne weiteres zu erwarten, daß aus derart empfindlichen Verbindungen durch Einbau von Alkylenoxideinheiten, vorzugsweise von Ethylenoxid, und Veresterung der

Poly-(thioetheralkohole) hochwirksame Stabilisatoren zugänglich werden, deren Handhabung keinerlei Probleme bereitet.

Die erfindungsgemäß vorzugsweise in Polypropylen und Polyethylen als Stabilisatoren einzusetzenden Ester sind neue, überwiegend kristalline Verbindungen, deren Charakterisierung durch Schmelzpunkte und spektroskopische Untersuchungen den angefügten Beispielen zu entnehmen ist. Solche kristallinen Verbindungen sind gegenüber flüssigen Stabilisatoren, wie erwähnt, bevorzugt, da ihre gleichmäßige Verteilung im Polymeren und der kontinuierliche Zusatz der notwendigen kleinen Mengen i. a. geringere Schwierigkeiten bereiten.

Die Herstellung der erfindungsgemäßen Ester kann nach an sich bekannten Verfahren durch Umsetzung reaktiver Säurederivate mit den durch Oxethylierung der erwähnten Ausgansstoffe zugänglichen polyfunktionellen Alkoholen erfolgen. Diese sollten mindestens zwei, vorzugsweise ≧ 2, Alkylenoxideinheiten enthalten, d. h. mindestens zwei Thioetherbrücken aufweisen, wobei diese Thioetherbrücken von ≧ 2 verschiedenen Thiolgruppen ausgehen. Zur Oxethylierung können die bekannten Verfahren des Standes der Technik (vgl. z. B. M. J. Schick, "Nonionic Surfactants", Vol. I, M. Dekker, New York (1967), S. 175 ff.; Houben-Weyl, "Methoden der organischen Chemie", Bd.VI/3 (1965) S. 461 ff.) angewandt werden, nach denen Alkylenoxide der Formel V

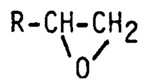

(R = H, $C_1$-$C_4$-Alkyl, Aryl)

an die entsprechenden Ausgangsstoffe der Formeln II bis IV angelagert werden. Anlagerungsprodukte von Ethylenoxid, d. h. R = H in Formel V, sind als Ausgangsmaterialien der erfindungsgemäßen Ester von 3-tert.Butyl- bzw. 3-tert.Butyl-5-alkyl-4-hydroxyphenyl-(alkan-) carbonsäuren der Formel I bevorzugt.

Ethylenoxidaddukte von Thiolen können nach dem Stand der Technik auch durch Umsetzung von Thiolen mit Ethylencarbonat (vgl. z. B. US-PS 2 448 767) bzw. aus geeigneten halogenhaltigen Vorstufen durch Umsetzung mit 2-Thioethanol ($HSCH_2CH_2OH$) unter Basenkatalyse nach

$$RX + HSCH_2CH_2OH \rightarrow RSCH_2CH_2OH + HX$$

gewonnen werden (vgl. z. B. Ullmann, Enzyklopädie der Techn. Chemie, Bd. 23 (1983), S. 186).

Ganz besonders bevorzugte Vorstufen sind Poly-(monooxethylate) von Polythiolaromaten bzw. Dithiolcycloalkanen, in denen sämtliche Thiolgruppen durch Einwirkung von Ethylenoxid in (2-Hydroxyethylthio-)substituenten überführt wurden. Derartige Poly-(2-hydroxyethylthio-)aromaten bzw. -cycloalkane sind nicht für alle den Formeln II bis IV entsprechenden Polythiole vorbeschrieben. Die Abtrennung der gewünschten Poly-(monooxethylate) von ggf. bei der Oxethylierung entstandenen Nebenprodukten, wie z. B. Polyglykolen oder höheren Oxethylaten, kann nach bekannten Verfahren des Standes der Technik, d. h. durch Kristallisation aus geeigneten Lösemitteln (wie Alkoholen oder Ethern), durch Säulenchromatographie oder - in Einzelfällen - durch Destillation unter vermindertem Druck erfolgen.

Die Herstellung der erfindungsgemäßen Ester ist besonders einfach, wenn die schwefelhaltigen Poly-(monooxethylate) mit Estern der 3-tert.Butyl- bzw. 3-tert.Butyl-5-alkyl-4-hydroxyphenyl-(alkan-)carbonsäuren der Formel I, vorzugsweise mit Estern der 3-(3,5-Di-tert.butyl-4-hydroxyphenyl-)propionsäure, die Alkoxygruppen mit ein bis vier C-Atomen enthalten, unter Freisetzung und Entfernung der leichter flüchtigen Alkohole umgeestert werden. Die Umesterung wird im allgemeinen bei erhöhter Temperatur, vorzugsweise unterhalb von 140 °C, durchgeführt. Die direkte Umsetzung von Polyol und Niedrigalkylestern ist häufig der wirtschaftlichste Weg, doch kann auch in Gegenwart inerter Löse- und/oder Schleppmittel zur Abtrennung des freiwerdenden Alkohols gearbeitet werden. Zur Beschleunigung der Umesterung werden (vorzugsweise basische oder neutrale) Katalysatoren nach dem Stand der Technik verwendet, wie z. B. Natriummethylat, Lithiumamid, Kalium-tert.butylat, Titantetrabutylat, Aluminiumtriisopropylat u. a. Diese werden im allgemeinen in Mengen von 0.1 bis 5 Gewichtsprozent, bezogen auf das Gewicht des Reaktionsgemisches, zugesetzt, wobei Mengen von 0.5 bis 1.5 Gewichtsprozent aus Gründen der Reaktionsgeschwindigkeit und der Kosten bevorzugt sind.

Zweckmäßigerweise wird die Umesterung unter Inertgas oder bei vermindertem Druck durchgeführt, um Oxidationen von Einsatz - bzw. Umsetzungsprodukten zu vermeiden und um die thermische Belastung des Reaktionsgemisches, vor allem in Gegenwart der Umesterungskatalysatoren, gering zu halten. Es ist von Vorteil, einen der Einsatzstoffe in 10- bis 20%igem molaren Überschuß über die erforderliche stöchiometrische Menge hinaus zu verwenden. Die Umsetzung wird unterbrochen, sobald durch Analyse des Reaktionsgemisches oder durch Wägung des abgetrennten Alkohols weitgehender (bzw. vollständiger) Verbrauch der im Unterschuß eingesetzten Komponente nachgewiesen ist. Dazu wird der Katalysator inaktiviert (d. h. bei basischen oder neutralen Katalysatoren z. B. durch Zusatz einer äquivalenten Säuremenge zerstört), das Reaktionsgemisch aufgearbeitet und die schwefelhaltigen Polyolester durch Umkristallisieren aus geeigneten Lösungsmitteln wie z. B. Alkoholen, Ethern oder aromatischen Kohlenwasserstoffen gereinigt. Anschließend wird die Konstitution der Verbindungen $^1$H-NMR-spektroskopisch an Hand der Lage und Intensität der verschiedenen Signale gesichert.

Die erfindungsgemäßen 3-tert.Butyl- bzw. 3-tert.Butyl-5-alkyl-4-hydroxyphenyl-(alkan-)carbonsäureester von Poly-(2-hydroxyethylthio-) bzw. Poly-(2-hydroxyethylthiomethyl-)aromaten sowie von Di-[2-hydroxyethylthio-(alkyl-)]cycloalkanen sind wertvolle Stabilisatoren für Polymere, vorzugsweise für (Co-)Polymerisate von Mono-und/oder Diolefinen, insbesondere von Polypropylen und Polyethylen. Sie können als Verarbeitungsstabilisatoren, aber auch als Langzeitstabilisatoren verwandt werden. Das Einmischen in die Polymeren kann nach bekannten Verfahren des Standes der Technik durch Vermischen der pulverförmigen Polymeren mit den Stabilisatoren oder mit einem Stabilisatorkonzentrat (in dem jeweiligen Polymeren) erfolgen. Der Stabilisatorzusatz kann aber auch in eine Suspension, Emulsion oder Lösung der Polymeren vor der Aufarbeitung eingebracht werden. Die Stabilisatoren werden in Mengen von ca. 0.02 bis 3 Gewichtsprozent des zu stabilisierenden Materials verwandt, doch schwankt die vom Fachmann leicht zu ermittelnde optimale Menge in Abhängigkeit vom zu stabilisierenden Polymeren und der Art der Beanspruchung.

Ein vorteilhafter Bereich liegt zwischen 0.05 und 2 Gewichsprozent Stabilisatorzusatz, ganz besonders günstig sind bei Polyolefinen Zusätze von 0,1 bis 1 Gewichtsprozent. Dabei können wahlweise einzelne Ester, aber auch Gemische mehrerer zugesetzt werden. Ferner können verschiedene weitere Zusatzstoffe wie z. B. organische schwefel-und/oder phosphorhaltige Verbindungen als Synergisten, aber auch nicht erfindungsgemäße Stabilisatoren, Weichmacher, Pigmente, UV-Stabilisatoren, Antistatika, Füllstoffe und/oder Verarbeitungshilfsmittel, wie Calciumstearat, zusätzlich in die Polymeren eingearbeitet werden.

Die erfindungsgemäße Stabilisierung von Polymeren, die im Falle der Polyolefine nach dem Spritzguß - bzw. Extrusionsverfahren zu vielseitig verwendbaren Fertigteilen verarbeitet werden können, wird durch die folgenden Beispiele näher erläutert.

Beispiel 1 (nicht erfindungsgemäß)

Herstellung von 1,4-Bis-(2′-hydroxyethylthiomethyl-)benzol

In einer sorgfältig getrockneten Apparatur aus einem 1000 ml-Dreihalskolben mit Rückflußkühler, Rührer, Tropftrichter mit Druckausgleich und Inertgaseinleitungsanschluß werden zunächst unter Inertgas 0.85 Mol (19,54 g) Natrium in 400 ml absolutem Ethanol gelöst. Anschließend werden 0.85 Mol (66.4 g) Thioethanol zugesetzt und zu dem dabei entstehenden Natrium-(2-hydroxyethylmercaptid) unter Rühren bei 20 °C 0.4 Mol (70 g) 1,4-Bis-(chlormethyl)-benzol, gelöst in 200 ml Ethanol, binnen ca. 10 Minuten zugetropft. Nach ca. fünfstündiger Nachreaktionszeit bei Siedetemperatur des Reaktionsgemisches wird das ausfallende Natriumchlorid nach Abkühlen der Lösung auf Raumtemperatur abfiltriert. Aus dem Filtrat kristallisiert nach Abziehen eines Teils des Lösungsmittels 1,4-Bis-(2′-hydroxyethylthiomethyl-)benzol in Form farbloser Kristalle aus.

Ausbeute: 81.2 g ( = 78 % d.Th.)
Schmelzpunkt: 91 °C (aus Ethanol)

Die Konstitution der Verbindung wird durch die Lage und Intensität der Signale des $^1$H-NMR-Spektrums bestätigt.

Beispiel 2

Herstellung des Di-[3-(3,5-ditert.butyl-4-hydroxyphenyl-)propionsäure-]esters des 1,4-Bis-(2′-hydroxyethylthiomethyl-)benzols

In einem mit Innenthermometer, Magnetrührer und Liebigkühler ausgerüsteten Dreihalskolben werden 0.29 Mol (75 g) 1,4-Bis-(2′-hydroxyethylthiomethyl-)benzol mit 0.62 Mol (181,3 g) 3-(3,5-Di-tert.butyl-4-hydroxyphenyl-)propionsäuremethylester und mit 0.076 Mol (4.1 g) Natriummethylat versetzt. Das Reaktionsgemisch wird in der vor der Reaktion getrockneten Apparatur unter Feuchtigkeitsausschluß und unter Inertgas auf 110 °C bis 115 °C erhitzt. Nach etwa einer Stunde Reaktionszeit wird der Druck stufenweise bis auf ca. 0.2 hPa (mbar) vermindert. Abdestillierendes Methanol wird in einer Kühlfalle kondensiert und zur

Abschätzung des erreichten Umsatzes nach Abschluß der Umesterung gewogen. Nahezu vollständiger Umsatz ist nach ca. 6 Stunden erreicht. Nach Abkühlen des Reaktionsgemisches unter Luftausschluß wird der Inhalt des Dreihalskolbens in ca. 250 bis 300 ml Toluol aufgenommen, eine dem Natriummethylatkatalysator annähernd äquivalente Menge Eisessig (5 % Überschuß) zugesetzt und die Toluollösung anschließend mit Natriumbicarbonatlösung und Wasser gewaschen. Nach Filtration und Trocknung wird zunächst Toluol bei vermindertem Druck entfernt, ehe aus dem zähen Destillationsrückstand unumgesetzter bzw. überschüssiger 3-(3,5-Di-tert.butyl-4-hydroxyphenyl-)propionsäuremethylester (Sdp. ca. 130 °C/0.05 hPa (mbar)) im Vakuum abgetrennt wird. Die nichtflüchtigen Anteile des Reaktionsgemisches bestehen überwiegend aus dem Bis-(3-(3,5-ditert.butyl-4- hydroxyphenyl-)propionsäure-)ester des 1,4-Bis-(2′-hydroxyethylthiomethyl-)benzols. Durch Zusatz einer kleinen Menge (ca. 180 ml) Diethylether läßt sich der Diester zur Kristallisation bringen. Das Rohprodukt wird durch Umkristallisieren aus Diethylether bis zur Schmelzpunktskonstanz gereinigt, die erreichte Reinheit dünnschichtchromatographisch (Abk. TLC) überprüft.

| | |
|---|---|
| Ausbeute: | 160 g (= 70.9% d.Th.) |
| Schmelzpunkt: | 95.5 bis 96.5 °C |
| Reinheit: | 92 % (lt. TLC) |

Die Konstitution der Verbindung wird durch Intensität und Lage der Signale des $^1$H-NMR-Spektrums bestätigt. (Vgl. Tabelle I, die nähere Einzelheiten über die (mit TMS (Tetramethylsilan) als innerem Standard überwiegend in $CDCl_3$ aufgenommenen) Spektren der erfindungsgemäßen Ester enthält.)

Beispiel 2.1

Das Beispiel 2 wird unter Verwendung von 0.15 Mol (38.8 g) 1,4-Bis-(2-hydroxyethylthiomethyl-)benzol, 0.33 Mol (96.5 g) 3-(3,5-Di-tert.butyl-4-hydroxyphenyl-)propionsäuremethylester und 0.01 Mol (3.4 g) Titantetrabutylat (anstelle von Natriummethylat) wiederholt. Nach 6 Stunden ist ein nahezu vollständiger Umsatz des eingesetzten Diols erreicht. Nach Aufarbeitung wird der Bis-(3-(3,5- ditert.butyl-4-hydroxyphenyl-)propionsäure-)ester des 1,4-Bis-(2′-hydroxyethylthiomethyl-)benzols in 75.1 % Ausbeute (87,8 g) erhalten.

| | |
|---|---|
| Schmelzpunkt: | 96 bis 96.5 °C (aus Diethylether) |
| Reinheit : | ≧ 95 % (lt. $^1$H-NMR) |

Beispiele 3 bis 9 (nicht erfindungsgemäß)

Herstellung verschiedener Poly-(2-hydroxyethylthiomethyl-)benzolverbindungen

Nach der im Beispiel 1 beschriebenen Verfahrensweise werden 0.5 bis 2.0 Mol Natrium-(2-hydroxyethylmercaptid) mit 1,2-Bis-(chlormethyl-) benzol (Beispiel 3), 1,4-Bis-(brommethyl-)2,3,5,6-tetrabrombenzol (Beispiel 4), 1,3,5-Trichlor-2,4,6-tris-(brommethyl-)benzol (Beispiel 5), 1,4-Dichlor-2,3,5,6-tetra-(brommethyl-)benzol (Beispiel 6), 1,4-Dibrom-2,3,5,6-tetra-(brommethyl-)benzol (Beispiel 7), Methyl-penta-(brommethyl-)benzol (Beispiel 8) und Hexa-(brommethyl-)benzol (Beispiel 9) zu den entsprechenden Poly-(2-hydroxyethylthiomethyl-)benzol-verbindungen umgesetzt. In allen Fällen wird ein geringer Überschuß (ca. 5 Mol-%) an Natrium-(2-hydroxyethylmercaptid) verwandt. Bei schwer in Ethanol löslichen Halogenmethylaromaten ist die Umsetzung in trockenem Diethylenglykoldimethylether durchzuführen und die Nachreaktionszeit nach Vermischen der Ausgangstoffe ggf. zu verlängern, um vollständigen Austausch aller Halogenatome zu erreichen. Ausfallendes Natriumhalogenid wird abfiltriert und die Poly-(2-hydroxyethylthiomethyl-)aromaten nach Einengen der Filtrate isoliert. Zur weiteren Reinigung sind die Polyole umzukristallisieren, ehe an Produkten mit konstanten Schmelzpunkten deren Konstitution mittels $^1$H-NMR-Analyse überprüft wird. Tabelle II enthält eine Aufstellung der synthetisierten, größtenteils nicht vorbeschriebenen Verbindungen mit den bei der Herstellung bzw. Umkristallisation benutzten Lösemitteln sowie mit den ermittelten Schmelzpunkten und Ausbeuten.

Beipiel 10

Herstellung des Bis-(3-(3,5-ditert.butyl-4-hydroxyphenyl-)-propionsäure-)esters des 1,2-Bis-(2′-hydroxyethylthiomethyl-)benzols

Nach der im Beispiel 2 beschriebenen Verfahrensweise werden 0.27 Mol (69.7 g) 1,2-Bis-(2′-hydroxyethylthiomethyl-)benzol (Smp. 10 °C), das nach Beispiel 3 hergestellt wurde, in Gegenwart von 0.15 Mol (8.26 g) Natriummethylat mit 0.57 Mol (166.7 g) 3-(3,5-Ditert.butyl-4-hydroxyphenyl-)propionsäuremethylester bei 115 °C binnen 5.5 Stunden umgeestert. Nach Aufarbeitung und Entfernen unumgesetzter bzw.

überschüssiger Ausgangsmaterialien unter vermindertem Druck wird ein zähes Öl erhalten, das bei längerem Abkühlen (auch unter Zusatz geringer Lösemittelmengen) nicht kristallisiert. Das $^{1}$H-NMR-Spektrum bestätigt die Konstitution des Di-esters. Reinheit: > 95 % (lt. $^{1}$H-NMR)

Beispiel 11

Herstellung des Bis-(3-(3,5-ditert.butyl-4-hydroxyphenyl-)-propionsäure-esters des 1,4-Bis-(2′-hydroxyethylthiomethyl-)-2,3,5,6-tetrabrombenzols

Nach der im Beispiel 2 beschriebenen Verfahrensweise werden 0.13 Mol (74,6 g) 1,4-Bis-(2′hydroxyethylthiomethyl-)-2,3,5,6-tetrabrombenzol (Smp. 170 bis 171 °C), das nach Beispiel 4 hergestellt wurde, mit 0.31 Mol (90.7 g) 3-(3,5-Ditert.butyl-4-hydroxyphenyl-)propionsäuremethylester in Gegenwart von 0.005 Mol (1,7 g) Titantetrabutylat, das anstelle von Natriummethylat als Katalysator verwandt wurde, bei 145 °C binnen 7,6 Stunden umgeestert. Die analoge Aufarbeitung liefert ein Rohprodukt, aus dem nach Umkristallisieren aus Ethanol der erfindungsgemäße Bis-ester erhalten wird.

| | |
|---|---|
| Ausbeute : | 126,5 g Bis-ester (= 88,5 % d.Th.) |
| Schmelzpunkt: | 109.5 °C (aus Ethanol) |
| Reinheit : | >95 % (lt. TLC) |

Die Konstitution der Verbindung wird durch die Lage und die Intensität der Signale des $^{1}$H-NMR-Spektrums bestätigt. (Einzelheiten vgl. Tabelle I).

Beispiel 12

Herstellung des Tris-(3-(3,5-ditert.butyl-4-hydroxyphenyl-)propionsäure-)esters des 1,3,5-Tris-(2′-hydroxyethylthiomethyl-)-2,4,6-trichlor-benzols

Nach Beispiel 5 erhaltenes 1,3,5-Tris-(2′-hydroxyethylthiomethyl-)-2,4,6-trichlorbenzol (Smp. 140 bis 141,5 °C) (0.08 Mol; 35 g) wird nach der im Beispiel 2 beschriebenen Verfahrensweise in Gegenwart von 0.005 Mol (1,7 g) Titantetrabutylat mit 0.24 Mol (70.9 g) 3-(3,5-Ditert.butyl-4-hydroxyphenyl-)propionsäuremethylester bei 135 °C binnen 5,5 Stunden unter Abziehen freiwerdenden Methanols bei vermindertem Druck umgeestert. Nach analoger Aufarbeitung und Entfernen unumgesetzter bzw. überschüssiger Ausgangsmaterialien wird ein viskoser Rückstand erhalten, der beim Abkühlen glasartig erstarrt. Er besteht aus dem erfindungsgemäßen Tris-ester. Dessen Konstitution wird durch Lage und Intensität der Signale des $^{1}$H-NMR-Spektrums bestätigt (vgl. Tabelle I).

| | |
|---|---|
| Ausbeute : | 71 g (=74,3 % d.Th.) Tris-ester |
| Schmelzpunkt: | 56 bis 61 °C |
| Reinheit: | ≧ 95 % (lt. $^{1}$H-NMR) |

Beispiel 13

Herstellung des Tetra-(3-(3,5-ditert.butyl-4-hydroxyphenyl-) propionsäure-)esters des 1,2,4,5-Tetra-(2′-hydroxyethylthiomethyl-)-3,6-dichlorbenzols

Nach der im Beispiel 2 beschriebenen Verfahrensweise werden 0.14 Mol (53,2 g) 1,2,4,5-Tetra-(2′-hydroxyethylthiomethyl-)-3,6-dichlorbenzol (Smp. 165 bis 170,5 °C), das nach Beispiel 6 hergestellt wurde, mit 0.61 Mol (177,2 g) 3-(3,5-Ditert.butyl-4-hydroxyphenyl-)propionsäuremethylester in Gegenwart von 0.02 Mol (6.4 g) Titantetrabutylat bei 135 bis 140 °C binnen 26 Stunden umgeestert. Nach Aufarbeitung und Entfernen unumgesetzter bzw. überschüssiger Ausgangsmaterialien unter vermindertem Druck wird der erfindungsgemäße Tetra-ester des schwefelhaltigen, tetrafunktionellen Alkohols erhalten. Seine Konstitution wird durch das $^{1}$H-NMR-Spektrum bestätigt. (Nähere Einzelheiten sind Tabelle I zu entnehmen).

| | |
|---|---|
| Ausbeute : | 162,4 g (= 78,3 % d. Th.) Tetra-ester |
| Schmelzpunkt: | 133 bis 136 °C (aus Ethanol) |
| Reinheit : | > 95 % (lt. $^{1}$H-NMR) |

Beispiel 14

Herstellung des Tetra-(3-(3,5-ditert.butyl-4-hydroxyphenyl-)propionsäure-)esters des 1,2,4,5-Tetra-(2-hydroxyethylthiomethyl-)-3,6-dibrombenzols

Analog zu Beispiel 2 werden 0.12 Mol (71,6 g) 1,2,4,5-Tetra-(2′-hydroxyethylthiomethyl-)-3,6-dibrombenzol (Smp. 172.5 bis 174 °C), das nach Beispiel 7 gewonnen wurde, in Gegenwart von 0.011 Mol (3,8 g) Titantetrabutylat mit 0.52 Mol (152,1 g) 3-(3,5-Ditert.butyl-4-hydroxyphenyl-)propionsäuremethylester binnen 22 Stunden bei 135 °C umgeestert. Nach analoger Aufarbeitung und Entfernen unumgesetzter bzw. überschüssiger Ausgangsmaterialien bleibt der erfindungsgemäße Tetra-ester zurück, der aus Dioxan umkristallisiert wird. Seine Konstitution wird $^1$H-NMR-spektroskopisch bewiesen (vgl. Tabelle I).

| | |
|---|---|
| Ausbeute : | 196,6 g (= 75,5 % d.Th.) Tetra-ester |
| Schmelzpunkt: | 140 bis 142 °C (aus Dioxan) |
| Reinheit : | > 95 % (lt. 1H-NMR) |

Beispiel 15

Herstellung des Penta-(3-(3,5-ditert.butyl-4-hydroxyphenyl-)propionsäure-)esters des Penta-(2′-hydroxyethylthiomethyl-)methylbenzols

Nach der im Beispiel 2 beschriebenen Verfahrensweise werden 0.06 Mol (34 g) Penta-(2′-hydroxyethylthiomethyl-)-methylbenzol (Smp. 127 bis 129 °C), das nach Beispiel 8 hergestellt wurde, in Gegenwart von 0.005 Mol (1,7 g) Titantetrabutylat mit 0.35 Mol (100,9 g) 3-(3,5-Ditert.butyl-4-hydroxyphenyl-)propionsäuremethylester bei 150 °C binnen 5,5 Stunden umgeestert. Nach analoger Aufarbeitung und Entfernung unumgesetzter bzw. überschüssiger Ausgangsmaterialien wird ein viskoser Rückstand erhalten, der beim Abkühlen glasartig erstarrt und der aus dem erfindungsgemäßen Penta-ester besteht. Seine Konstitution wird durch das $^1$H-NMR- Spektrum bestätigt. (Vgl. Tabelle I hinsichtlich näherer Einzelheiten.)

| | |
|---|---|
| Ausbeute : | 115,5 g (= 78,2 % d. Th.) Penta-ester |
| Schmelzpunkt: | 55 bis 56,5 °C |
| Reinheit : | > 95 % (lt. TLC) |

Beispiel 16

Herstellung des Hexa-(3-(3,5-ditert.butyl-4-hydroxyphenyl-)propionsäure-)esters des Hexa-(2-hydroxyethylthiomethyl-)benzols

Nach der im Beispiel 2 beschriebenen Verfahrensweise werden 0.028 Mol (17,3 g) Hexa-(2-hydroxyethylthiomethyl-)benzol (Smp. 125 bis 126.5 °C), das nach Beispiel 9 erhalten wurde, mit 0.18 Mol (51,6 g) 3-(3,5-Ditert.butyl-4-hydroxyphenyl-)propionsäuremethylester binnen 8 Stunden bei 134 °C in Gegenwart von 0.016 Mol (0.9 g) Natriummethylat umgeestert. Nach analoger Aufarbeitung und Entfernen unumgesetzter bzw. überschüssiger Ausgangsmaterialien wird der erfindungsgemäße Hexa-ester isoliert. Das $^1$H-NMR-Spektrum bestätigt dessen Konstitution (vgl. Tabelle I).

| | |
|---|---|
| Ausbeute : | 16.2 g (= 26.5 % d.Th.) Hexa-ester |
| Schmelzpunkt: | 89,5 bis 91,5 °C (aus Methanol) |
| Reinheit : | ca. 93 % (lt. TLC) |

Beispiel 17 (nicht erfindungsgemäß)

Herstellung von Bis-2,9-(2′-hydroxyethylthio-)-para-menthan

Nach bekannten Verfahren zur Oxethylierung von Mercaptanen (vgl. z. B. M. J. Schick, loc. cit.) wird Dipentendimercaptan (2,9-para- Methandithiol) in Gegenwart katalytischer Mengen Natriumhydroxid mit Ethylenoxid umgesetzt. Die Reaktion wird abgebrochen, sobald die zwei Thiolgruppen entsprechende Menge Ethylenoxid aufgenommen ist. Nach Abkühlen, Neutralisieren des eingesetzten Katalysators mit einer äquivalenten Menge Eisessig, Entfernen gebildeten Natriumacetats und Abziehen leichter flüchtiger Bestandteile bei einem Druck von weniger als 0.1 hPa (mbar) bleibt ein viskoses Öl zurück, aus dem sich das gewünschte schwefelhaltige Diol durch fraktionierte Destillation (Sdp 188 °C/0.1 hPa (mbar) abtrennen läßt. Das $^1$H-NMR-Spektrum bestätigt die Konstitution der Verbindung.

Beispiel 18

Herstellung des Bis-(3-(3,5-ditert.butyl-4-hydroxyphenyl-)-propionsäure-)esters des Bis-2,9-(2′-

hydroxyethylthio-)para-menthans

Nach der im Beispiel 2 beschriebenen Verfahrensweise werden 0.31 Mol (90,7 g) Dipentendimercaptan-bis-(monooxethylat) [Bis-2,9-(2′-hydroxyethylthio-)menthan], das nach Beispiel 17 hergestellt wurde, mit 0.67 Mol (196 g) 3-(3,5-Ditert.butyl-4-hydroxyphenyl-)propionsäuremethylester in Gegenwart von 0.01 Mol (3.4 g) Titan-tetrabutylat bei 120 °C binnen 16 Stunden umgeestert, wobei freiwerdendes Methanol bei vermindertem Druck abgezogen wird. Nach analoger Aufarbeitung wie im Beispiel 2 und Entfernen unumgesetzter bzw. überschüssiger Ausgangsmaterialien (bei 0.03 hPa (mbar)) bleibt der erfindungsgemäße Di-ester als gelbliches Öl zurück, das weder durch längeres Kühlen noch durch Zusatz kleiner Mengen verschiedener Lösemittel zu kristallisieren ist. Die Konstitution des Di-esters wird durch das $^1$H-NMR-Spektrum bestätigt. (Nähere Einzelheiten sind Tabelle I zu entnehmen). Offensichtlich ist die Kristallisationsverzögerung auf das Vorliegen eines Gemisches von Konformeren zurückzuführen, da die Reinheit des Bis-esters nach dem $^1$H-NMR-Spektrum über > 90% betragen sollte.

Beispiele 19 bis 28
(Beispiele 26 bis 28 nicht erfindungsgemäß)

Stabilisierung von Polypropylen mit erfindungsgemäßen Stabilisatoren (Beispiele 19 bis 25) und mit Stabilisatoren nach dem Stand der Technik

2 kg Polypropylen-Pulver werden mit den in Tabelle II angegebenen Mengen an Stabilisator (erfindungsgemäße Ester nach den Beispielen 2 sowie 11 bis 16 bzw. Stabilisatoren nach dem Stand der Technik (in den Vergleichsbeispielen 26 bis 28)), an Calciumstearat (0.1 Gewichtsprozent) als Verarbeitungshilfsmittel sowie ggf. an Bis-(octadecyl-)thiodipropionsäureester und an Tris-(2.4-ditert.butylphenyl-)phosphit versetzt und in einem Mischaggregat (z. B. Fluid-Mischer der Fa. Papenmeier) bei Raumtemperatur vermischt. Die erhaltenen Pulvermischungen werden bei 100 Upm und maximal 230 °C mit einem Extruder (Fa.Tröster, d = 30 mm, l = 20 d) extrudiert und anschließend granuliert. Das Granulat wird bei 210 °C zu 1mm dicken Platten verpreßt und aus diesen Polyolefinstreifen mit den Maßen 1 × 10 × 100mm gestanzt. Letztere werden in einer geeigneten Vorrichtung bei 145 °C freistehend in einem Umlufttrockenschrank unter Luftzutritt solange gelagert, bis als Zeichen eintretender Versprödung ein Zerbröseln der Prüfkörper bzw. Rißbildung an diesen zu beobachten ist. Tabelle III enthält die Ergebnisse.

An der bei 145 °C ermittelten Beständigkeit stabilisierter Polypropylenproben erkennt man, daß die Wirkung der erfindungsgemäß einzuarbeitenden Ester i.a. das Niveau von Proben erreicht oder übertrifft, bei denen Stabilisatoren nach dem Stand der Technik zugesetzt wurden.

Beispiele 29 bis 38
(Beispiele 36 bis 38 nicht erfindungsgemäß)

Prüfung stabilisierter Polyethylenproben auf Farbveränderungen

Analog den Beispielen 19 bis 26 mit 0.1 Gewichtsprozent verschiedener Stabilisatoren und stets mit 0.1 Gewichtsprozent Calciumstearat versehene Polyethylengranulate wurden bei 210 °C zu Platten (mit den Maßen 4×10×100mm) verpreßt. Unterwirft man diese einer 28tägigen Alterung bei 100 °C und beurteilt anschließend die verschiedenen Proben hinsichtlich eingetretener Farbveränderungen im Vergleich zu analog hergestellten und gealterten Proben, die Stabilisatoren nach dem Stand der Technik [(Mono-, Bis- bzw. Tetra-(3,(3,5-ditert.butyl-4-hydroxyphenyl-)propionsäure-)ester des Octadecanols (Beispiel 36), des Thiodiethanols (Beispiel 37) bzw. des Pentaerythrits (Beispiel 38)] enthalten, so erhält man die in Spalte 5 der Tabelle III wiedergegebenen, visuell ermittelten Farbeindrücke.

Man erkennt an den Prüfergebnissen, daß mit der erfindungsgemäßen Stabilisierung Vorteile gegenüber dem Stand der Technik, wie er für schwefelhaltige Stabilisatoren durch das Beispiel 37 dargestellt wird, zu erreichen sind, da bei den Beispielen 29 bis 35 ausnahmslos deutlich geringere Verfärbungen beobachtet werden, sodaß sogar das nach dem Stand der Technik durch schwefelfreie Stabilisatoren vorgegebene Werteniveau annähernd erreicht wird.

Beispiele 39 bis 48
(Beispiele 46 bis 48 nicht erfindungsgemäß)

Nach den Beispielen 19 bis 25 sowie 26 und 28 hergestelltes, stabilisiertes Polypropylenpulver wurde

bei maximal 270 °C auf einem Extrusimeter (Fa. Göpfert; d = 20 mm, l = 20 d; 30 Upm) mehrfach extrudiert. An den erhaltenen Granulaten wurden bei 190 °C $I_5$-Werte als Maß für Veränderungen des Molekulargewichts bestimmt. Die ermittelten Werte sind in Tabelle IV zusammengefaßt.

Man erkennt an den ermittelten Werten, daß mit den erfindungsgemäßen Stabilisatoren bereits ohne Zusatz von Synergisten häufig eine bessere, mindestens aber eine ebenso gute Verarbeitungsstabilität wie mit den Stabilisatoren nach dem Stand der Technik erreicht werden kann. Bei Anwesenheit zusätzlicher Synergisten werden die Unterschiede zum Stand der Technik geringer.

Beispiel 49

Herstellung von 3-(3,5-Ditert.-butyl-4-hydroxyphenyl-)-propionsäureestern des 1,4-Dihydroxy-2,3,5,6-tetra-(2'-hydroxyethylthio-)benzols

Nach der im Beispiel 2 beschriebenen Verfahrensweise werden 0,07 Mol (29 g) 1,4-Dihydroxy-2,3,5,6-tetra-(2'-hydroxyethylthio-)benzol, das (nach M. Kulka, Can. J. Chem. 40 (1962) 1238) durch Umsetzung von Chloranil mit Thioethanol zugänglich ist, in Gegenwart von 0,34 Mol (18,4 g) Natriummethylat mit 0,38 Mol (111,1 g) 3-(3,5-Ditert.butyl-4-hydroxyphenyl-)propionsäuremethylester bei 120 °C binnen 6 Stunden umgeestert. Nach analoger Aufarbeitung und Entfernung unumgesetzter bzw. überschüssiger Ausgangsmaterialien wird ein beim Abkühlen erstarrender Rückstand erhalten, aus dem durch mehrfaches Umkristallisieren aus Toluol schwerer lösliche Verunreinigungen abgetrennt werden. Der nach Abziehen des Toluols verbleibende feste Rückstand besteht aus einem Gemisch verschiedener 3-(3,5-Ditert.butyl-4-hydroxyphenyl-)propionsäureester des 1,4-Dihydroxy-2,3,5,6-tetra-(2'-hydroxyethylthio-)benzols, wobei der Tetra-ester überwiegt. Das $^1H$-NMR-Spektrum (gemessen in $CDCl_3$ gegen TMS (intern) bestätigt die angenommene Konstitution aufgrund der Lage der Resonanzsignale (bei 7,0 bis 7,2 ppm; 5,1 ppm, 4,15 bis 4,3 ppm; 3,0 bis 3,5 ppm; 2,5 bis 2,7 ppm; 2,3 ppm und 1,43 ppm).

Tabelle I

$^{1}$H-NMR-Spektren von 3-(3,5-Ditert.butyl-4-hydroxyphenyl-)propionsäureestern von Oxethylaten verschiedener Polythiolaromaten und Dithiolcycloalkane

| Ester (Alkohol) nach Beispiel Nr. | Resonanzlinien der $^{1}$H-NMR-Spektren* [in ppm] (Zuordnung lt. Strukturformel*** der erfindungsgemäßen Ester) | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| 2 (1) | 6,98 (4) | 5,07 (2) | 4,20 (4) | 2,63 (4) | 2,87 (4) | 2,63 (4) | 1,42 (36) | 7,26 (4) | 3,72 (4) | | | |
| 10 (3) | 6,99 (4) | 5,08 (2) | 4,21 (4) | 2,64 (4) | 2,88 (4) | 2,64 (4) | 1,43 (36) | 7,23 (2) | 3,92 (4) | | | 7,18 (2) |
| 11 (4) | 6,98 (4) | 5,07 (2) | 4,36 (4) | 2,88 (8) | | 2,62 (4) | 1,43 (36) | | 4,31 (4) | | | |
| 12 (5) | 6,98 (6) | 5,08 (3) | 4,28 (6) | 2,83 (12) | | 2,63 (6) | 1,42 (54) | | 4,10 (6) | | | |
| 13 (6)** | 6,90 (8) | 6,70 (4) | 4,23 (8) | 2,86 (8) | 2,75 (8) | 2,57 (8) | 1,33 (72) | | 4,08 (8) | | | |
| 14 (7) | 6,98 (8) | 5,13 (8) | 4,33 (8) | 2,88 (12) | | 2,63 (8) | 1,42 (72) | | 4,26 (8) | | | |
| 15 (8) | 6,98 (10) | 5,07 (5) | 4,37 (10) | 2,90 (20) | | 2,65 (10) | 1,42 (90) | | 4,13 (6) | 3,95 (4) | 2,47 (3) | |
| 16 (9) | 6,98 (12) | 5,05 (6) | 4,37 (12) | 2,87 (24) | | 2,63 (12) | 1,40 (108) | | 4,12 (12) | | | |
| 18 (17) | 6,98 (4) | 5,10 (2) | 4,20 (4) | 2,68/2,86 (6) | | 2,61 (4) | 1,43 | | 2,98 (1) | 2,39 (1) | 0,8-2,1 | |

<u>Erläuterungen zu Tabelle I</u>

* gemessen in $CDCl_3$ gegen TMS (intern), Konz. ca. 20 Vol-%; angegeben sind die Signallagen in ppm (mit TMS = 0 ppm) und die Anzahl der zugehörigen H-Atome (jeweils Klammern)

** gemessen in d-DMSO (Deuterodimethylsulfoxid)

*** Zuordnung der Signale:

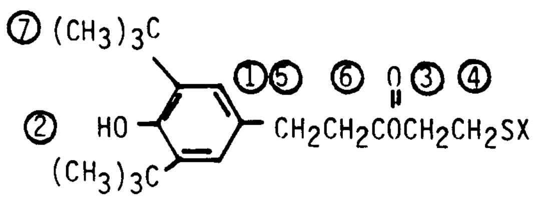

mit X = $-(CH_2)_n-$ 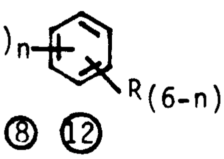

und R = H ⑧ ⑫

$-(CH_2)-$ ⑨

(für R = Cl,Br)

$-(CH_2)-$ ⑨⑩

(für R = $CH_3$ ⑪ )

bzw. mit X = 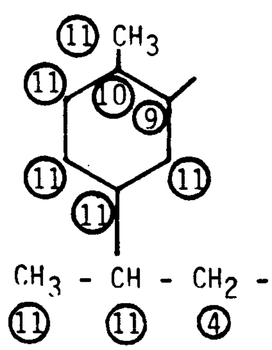

Tabelle II: Zur Herstellung erfindungsgemäßer Ester eingesetzte schwefelhaltige Polyole (vgl. nicht erfindungsgemäße Beispiele 3 bis 9 und 17)

| Polyol nach Beispiel Nr. | Formel des Polyols (mit $X=SCH_2CH_2OH$) | Ausgangsprodukt bei der Polyolsynthese | Herstellung der Polyole | | | |
|---|---|---|---|---|---|---|
| | | | Ausbeute (% d.Th.) | Schmelzpunkt (° C) | Lösemittel zur Umkristallisation | Lösemittel bei Herstellung |
| 1 | 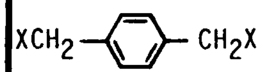 | X = Cl | 84.5 | 90-91.5 | Ethanol | Ethanol |
| 3 | 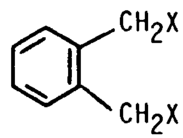 | X = Cl | 66.2 | 10° | | " |
| 4 | 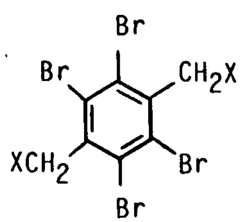 | X = Br | 77.0 | 176-177 | Diglyme | " |
| 5 | 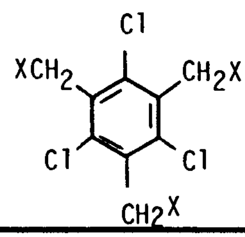 | X = Br | 63.3 | 140-141.5 | Ethanol | Ethanol/Diglyme |

Fortsetzung Tabelle II:

| Polyol nach Beispiel Nr. | Formel des Polyols (mit X=$SCH_2CH_2OH$) | Ausgangsprodukt bei der Polyolsynthese | Herstellung der Polyole | | | |
|---|---|---|---|---|---|---|
| | | | Ausbeute (% d.Th.) | Schmelzpunkt (° C) | Lösemittel zur Umkristallisation | Lösemittel bei Herstellung |
| 6 | 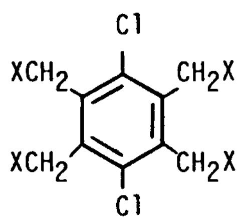 | X = Br | 90.4 | 176-177 | Diglyme | Ethanol |
| 7 | 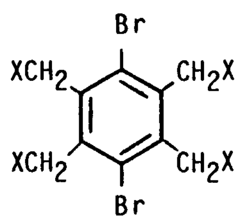 | X = Br | 81.0 | 179-180 | Diglyme | Ethanol |
| 8 | 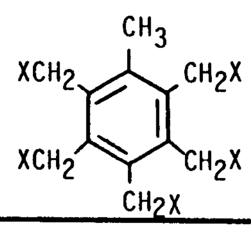 | X = Br | 62.3 | 127-129 | Ethanol | " |

Fortsetzung Tabelle II:

| Polyol nach Beispiel Nr. | Formel des Polyols (mit X=$SCH_2CH_2OH$) | Ausgangsprodukt bei der Polyolsynthese | Herstellung der Polyole | | | |
|---|---|---|---|---|---|---|
| | | | Ausbeute (% d.Th.) | Schmelzpunkt (° C) | Lösemittel zur Umkristallisation | Lösemittel bei Herstellung |
| 9 | 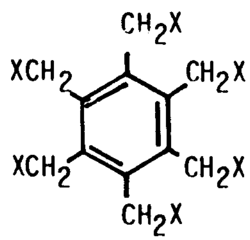 | X = Br | 31.2 | 125-126.5 | Ethanol | Ethanol |
| 17 | 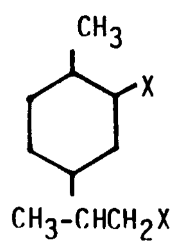 | X = SH | | flüssig (Sdp. 188°/ 0.1) | | |

Tabelle III: Alterungsversuche bei 145 °C bzw. 100 °C unter Zusatz erfindungsgemäßer Stabilisatoren zu Polypropylen bzw. Polyethylen

| Beispiel Nr. | Stabilisator nach Beispiel Nr. | Formel der Stabilisatoren | Alterung bei 145 °C (Tage) A* | B* | C* | Farbbeurteilung ** (Beispiele 29-38) |
|---|---|---|---|---|---|---|
| 19(29) | 2 | 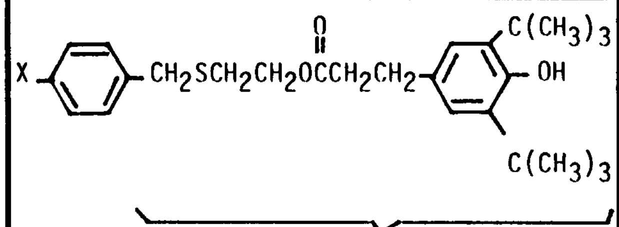 Abb. X | 22 | 38 | 40 | 0 |
| 20(30) | 11 | 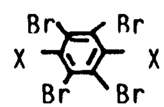 | 27 | 48 | 52 | 0 |
| 21(31) | 12 | 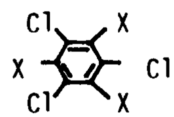 | 48 | 73 | >84 | 0 |
| 22(32) | 13 | 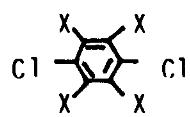 | 29 | 63 | 70 | 0 |

Fortsetzung Tabelle III:

| Beispiel Nr. | Stabilisator nach Beispiel Nr. | Formel der Stabilisatoren | Alterung bei 145 °C (Tage) A* | B* | C* | Farbbeurteilung ** (Beispiele 29-38) |
|---|---|---|---|---|---|---|
| 23(33) | 14 | 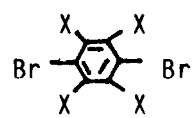 | 26 | 65 | 62 | 0 |
| 24(34) | 15 | 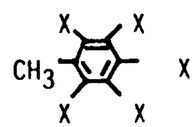 | 23 | 51 | 50 | 0 |
| 25(35) | 16 | 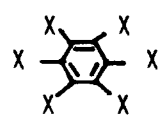 | 11 | 31 | 33 | 0 |

Fortsetzung Tabelle III:

| Beispiel Nr. | Stabilisator nach Beispiel Nr. | Formel der Stabilisatoren | Alterung bei 145 °C (Tage) A* | B* | C* | Farbbeurteilung ** (Beispiele 29-38) |
|---|---|---|---|---|---|---|
| 26(36) | Vergleichsversuch nach dem Stand der Technik | 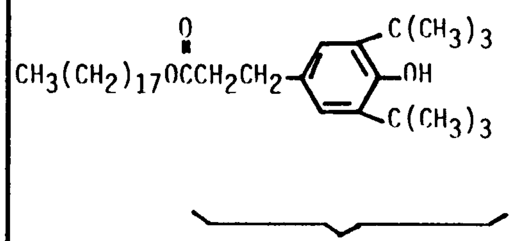 Abb. Y | 3 | 10 | 13 | 0 |
| 27(37) | " | $Y\text{-}OCH_2CH_2SCH_2CH_2O\text{-}Y$ | 15 | 24 | 32 | -- |
| 28(38) | " | $C(CH_2O\text{-}Y)_4$ | 50 | 74 | 81 | 0 |

Erläuterungen zu Tabelle II:

* Abkürzungen:

A = 0.1 Gew.-% Stabilisatorzusatz zu Polypropylen

B = wie A und zusätzlich 0.1 Gew.-% Bis-(octadecyl-)thiodipropionsäureester

C = wie B und zusätzlich 0.1 Gew.-% Tris-(2,4-ditert.butylphenyl-)phosphit

** Farbbeurteilung stabilisierter Polyethylenproben bei 100 °C nach vierwöchiger Alterung. (Die Angaben sind stets auf den Vergleichsversuch (Beispiel 38) bezogen.)

0 : ebensogut wie Beispiel 38

(-): etwas schlechter als Beispiel 38

-: schlechter als Beispiel 38

--: wesentlich schlechter als Beispiel 38

Tabelle IV: Mehrfachextrusion stabilisierter Polypropylenproben

| Beispiel Nr. | Polypropylen nach Beispiel Nr. | MFI-Werte (g/10 Min) nach Mehrfachextrusion | | | | | | | | | | | |
|---|---|---|---|---|---|---|---|---|---|---|---|---|---|
| | | ohne Zusatz von Synergisten* | | | | | | mit Zusatz von Synergisten** | | | | | |
| | | 0x | 1x | 2x | 3x | 4x | 5x | 0x | 1x | 2x | 3x | 4x | 5x |
| 39 | 19 | 3.0 | 4.6 | 7.3 | 11.9 | 13.5 | 17.5 | | | | | | |
| 40 | 20 | 3.4 | 7.4 | 10.2 | 14.7 | 19.6 | 24.4 | 3.2 | 4.0 | 5.1 | 6.0 | 7.1 | 8.5 |
| 41 | 21 | 3.2 | 6.0 | 8.9 | 12.9 | 17.1 | 22.2 | 3.1 | 4.2 | 5.1 | 6.0 | 7.5 | 8.3 |
| 42 | 22 | 2.7 | 6.1 | 8.7 | 11.7 | 15.6 | 19.6 | 2.8 | 4.1 | 5.2 | 5.7 | 7.1 | 8.7 |
| 43 | 23 | 3.4 | 7.0 | 9.0 | 12.8 | 16.6 | 22.5 | 3.4 | 4.4 | 5.3 | 6.0 | 7.1 | 8.5 |
| 44 | 24 | 3.0 | 5.5 | 8.1 | 11.3 | 14.7 | 17.8 | 2.8 | 4.4 | 5.0 | 6.2 | 7.3 | 9.0 |
| 45 | 25 | 2.8 | 5.6 | 8.0 | 11.5 | 14.9 | 16.7 | 3.2 | 4.2 | 5.0 | 5.9 | 7.0 | 8.3 |

Fortsetzung Tabelle IV:

| Beispiel Nr. | Polypropylen nach Beispiel Nr. | MFI-Werte (g/10 Min) nach Mehrfachextrusion | | | | | | | | | | | |
|---|---|---|---|---|---|---|---|---|---|---|---|---|---|
| | | ohne Zusatz von Synergisten* | | | | | | mit Zusatz von Synergisten** | | | | | |
| | | 0x | 1x | 2x | 3x | 4x | 5x | 0x | 1x | 2x | 3x | 4x | 5x |
| (Vergleichsversuche) | | | | | | | | | | | | | |
| 46 | 26 | 3.7 | 11.2 | 18.9 | 27.4 | 37.8 | 52.4 | 3.0 | 3.9 | 5.3 | 6.3 | 7.7 | 9.3 |
| 48 | 28 | 2.8 | 5.8 | 8.9 | 12.6 | 17.6 | 20.4 | 3.0 | 3.8 | 4.4 | 5.3 | 6.2 | 7.2 |

* Proben mit 0.1 Gew.-% Stabilisator und 0.1 Gew.-% Calciumstearat

** Proben enthalten zusätzlich 0.1 Gew.-% Bis-(octadeyl-)thiodipropionsäureester und 0.1 Gew.-% Tris-(2,4-ditert.butylphenyl-)phoshit

Beispiele 50 und 51
(nicht erfindungsgemäß)

Nach der im Beispiel 1 beschriebenen Verfahrensweise werden 1,3,5-Tribrom-2,4,6-tris-(brommethyl-

)benzol (Beispiel 50) bzw. 1,3,5-Trimethyl-2,4,6-tris(brommethyl-)benzol (Beispiel 51) zu den entsprechenden Tris-(2-hydroxyethylthiomethyl-)benzolverbindungen umgesetzt. Die Konstitution der erhaltenen Triole wurde durch $^{1}$H-NMR-Spektroskopie bestätigt. Die Verbindungen haben Schmelzpunkte von 161,5 °C (Beispiel 50) bzw. 163 °C (Beispiel 51).

Beispiele 52 und 53

Nach den Angaben des Beispiels 2 werden die nach Beispiel 50 bzw. 51 hergestellten Triole unter Katalyse mit Titantetrabutylat in 14 bis 21 Stunden mit 3-(3,5-Di-tert.butyl-4-hydroxyphenyl-)propionsäuremethylester umgeestert. Die erhaltenen Ester zeigten Schmelzpunkte von 53 bis 55 °C (Beispiel 52) bzw. 99 bis 100 °C (Beispiel 53). Ihre Konstitution wird durch die $^{1}$H-NMR-Spektren bestätigt. Resonanzlinien in ppm/Zuordnung analog zu Strukturformel Tabelle 1 ***.

1: 7,00 (6) 2: 5,06 ( 3) 3: 4,29 (6) 4 + 5: 2,89 (12)
6: 2,64 (6) 7: 1,43 (54) 9: 4,32 (6) (Beispiel 52)
1: 6,99 (6) 2: 5,08 ( 3) 3: 4,31 (6) 4: 2,79 (6) 5: 2,89 (6)
6: 2,63 (6) 7: 1,43 (54) 9: 3,84 (6) 11: 2,46 (9)

**Patentansprüche**

1. Ester von 3-tert.Butyl- bzw. 3-tert.Butyl-5-alkyl-4-hydroxyphenyl-(alkan-)carbonsäuren der Formel I

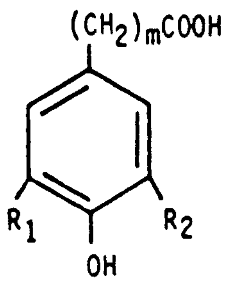

(I)

mit
$R_1$ = $-C(CH_3)_3$,
$R_2$ = H,$C_1$- bis $C_4$-Alkyl, vorzugsweise $-C(CH_3)_3$,
m = 0 bis 4, vorzugsweise 2,
mit Oxethylaten von Polythiolaromaten der allgemeinen Formeln II und III, vorzugsweise der Formel II, mit 2 bis 6 Thiolgruppen

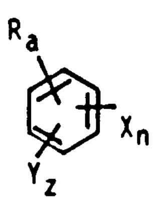

(II)

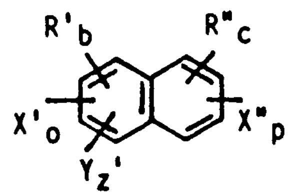

(III)

sowie mit Oxethylaten von Dithiolcycloalkanen der allgemeinen Formel IV, die maximal 6 Alkylenoxideinheiten enthalten,

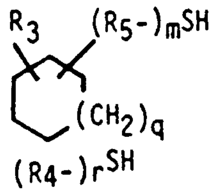

(IV)

mit $X = (CH_2)_m\ SH$, $X' = (CH_2)_r\ SH$, $X'' = (CH_2)_s\ SH$ wobei m,r,s = 0 oder 1 sein können

R,R',R" = H, Alkyl, Halogen, Hydroxyl
$R_3$ = H, $C_1$- bis $C_4$-Alkyl,
$R_4$,$R_5$ = $C_2$- bis $C_4$-Alkylen,
q = 0 bis 7, vorzugsweise 1,

Y = 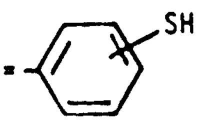

n = 2 bis 6 für z bzw. z' = 0,
n = 1 bis 5 für z bzw. z' ≠ 0,
o+p = n
wobei
$0 \leqq a, z \leqq a + Z \leqq 6\text{-}n$ (in Formel II)
$0 \leqq b, c, z' \leqq 4 \leqq 8\text{-}n$ (in Formel III)
sein können und die Anwesenheit einer einzelnen OH-Gruppe in Formel II ausgeschlossen ist.

**2.** Ester der 3-(3,5-Ditert.butyl-4-hydroxyphenyl-)propionsäure der Formel Ia

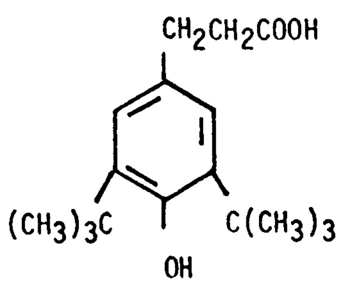

(Ia)

mit Oxethylaten von Polythiolaromaten der allgemeinen Formeln II und III mit 2 bis 6 Thiolgruppen bzw. mit Oxethylaten von Dithiolcycloalkanen der allgemeinen Formel IV.

**3.** Ester nach Anspruch 1 und 2
mit Oxethylaten von Polythiolaromaten der Formel II mit 2 bis 4 Thiolgruppen.

**4.** Ester nach Anspruch 1 und 2

mit Oxethylaten von Polythiolaromaten der Formeln II und III, die bevorzugt eine Ethylenoxideinheit pro Thiolgruppe der Polythiolaromaten als 2-Hydroxyethylthiogruppe enthalten.

**5.** Ester nach Anspruch 1 und 2
mit Bis-(monooxethylaten) des 1,4- bzw. 1,2-Bis-(thiomethyl-)benzols und des 1,4-Bis-(thiomethyl-)-2,3,5,6-tetrabrombenzols, mit dem Tris-(monooxethylat) des 1,3,5-Tris-(thiomethyl-)-2,4,6-trichlorbenzols, mit Tetra-(monooxethylaten) des 1,4,-Dichlor-2,3,5,6-tetra-(thiomethyl-)benzols und des 1,4-Dibrom-2,3,5,6-tetra-(thiomethyl-)benzols, mit dem Penta-(monooxethylat) des Penta-(thiomethyl-)toluols und mit dem Hexa-(monooxethylat) des Hexa-(thiomethyl-)benzols (= Hexakis-(2-hydroxyethylthiomethyl-) benzol) sowie mit dem Bis-(monooxethylat) des Dipentendimercaptans ($\hat{=}$ 2,9-para-Methandithiol).

**6.** Verfahren zur Herstellung der Ester nach einem der Ansprüche 1 bis 5,
dadurch gekennzeichnet,
daß man die 3-tert.Butyl- bzw. 3-tert.Butyl-5-alkyl-4-hydroxyphenyl-(alkan)carbonsäuren bzw. deren Derivate mit den Oxethylaten der Polythiolaromaten oder Dithiolcycloalkane verestert oder in Gegenwart von an sich bekannten alkalischen oder neutralen Katalysatoren umestert.

**7.** Verwendung der Ester nach einem der Ansprüche 1 bis 5 zur Stabilisierung von Polymeren.

**8.** Verwendung der Ester nach einem der Ansprüche 1 bis 5 zur Stabilisisierung von Polyolefinen.

**9.** Verwendung der Ester nach einem der Ansprüche 1 bis 5 zur Stabilisierung von Polyethylen und Polypropylen.

**10.** Verwendung der Ester nach einem der Ansprüche 1 bis 5
in einer Konzentration von 0,02 bis 3 Gewichtsprozent, vorzugsweise 0,05 bis 2 Gewichtsprozent, bezogen auf das Polymere, zur Stabilisierung dieser Polymeren.

## Claims

**1.** Esters of 3-tert.butyl- and 3-tert.butyl-5-alkyl-4-hydroxyphenyl-(alkane-)carboxylic acids having the Formula I

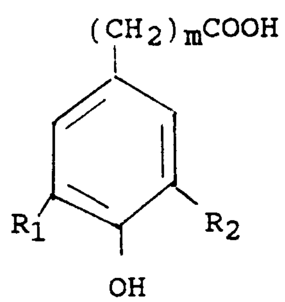

(I)

with
$R_1$ = $-C(CH_3)_3$,
$R_2$ = H, $C_1$ or $C_4$ alkyl, preferably $-C(CH_3)_3$,
m = 0 to 4, preferably 2,
with oxethylates of polythiol aromatics having the general Formulae II and III, preferably Formula II, with 2 to 6 thiol groups

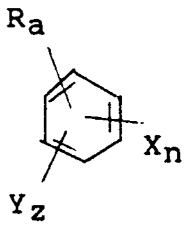 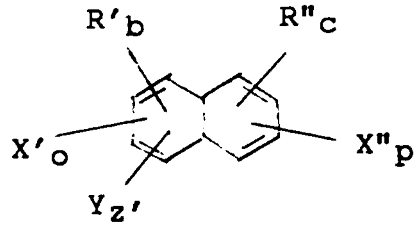

(II) (III)

and with oxethylates of dithiol cycloalkanes having the general Formula IV, which contain a maximum of 6 alkylene oxide units,

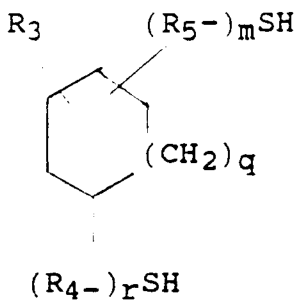

(IV)

with $X = (CH_2)_m SH$, $X' = (CH_2)_r SH$, $X'' = (CH_2)_s SH$, where $m, r, s = 0$ or $1$ is possible

| | |
|---|---|
| R,R',R" = | H, alkyl, halogen, hydroxyl, |
| $R_3$ = | H, $C_1$ to $C_4$ alkyl, |
| $R_4$, $R_5$ = | $C_2$ to $C_4$ alkylene, |
| q = | 0 to 7, preferably 1, |

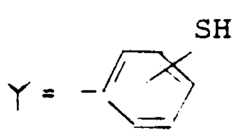

| | |
|---|---|
| n = | 2 to 6 for z and z' = 0, |
| n = | 1 to 5 for z and z' ≠ 0, |
| o+p = | n |

where
$0 \leqq a, z \leqq a + z \leqq 6\text{-}n$ (in Formula II)

0 ≦ b, c, z' ≦ 4 ≦ 8-n (in Formula III)
are possible and it is impossible for a single OH group to be present in Formula II

2. Esters of 3-(3,5-ditert.butyl-4-hydroxyphenyl-)propionic acid having the Formula Ia

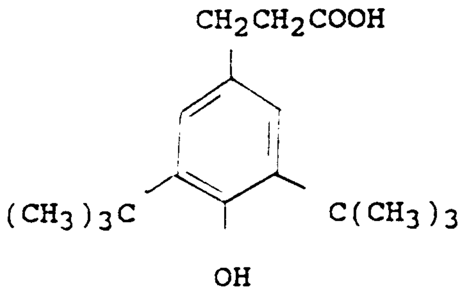

(Ia)

with oxethylates of polythiol aromatics having the general Formulae II and III with 2 to 6 thiol groups and with oxethylates of dithiol cycloalkanes having the general Formula IV.

3. Esters according to claims 1 and 2
with oxethylates of polythiol aromatics having the Formula II with 2 to 4 thiol groups.

4. Esters according to claims 1 and 2
with oxethylates of polythiol aromatics having the Formulae II and III, which preferably contain 1 ethylene oxide unit per thiol group of the polythiol aromatics as a 2-hydroxyethyl thiol group.

5. Esters according to claims 1 and 2
with bis-(monooxethylates) of 1,4- and 1,2-bis-(thiomethyl-)benzene and 1,4-bis-(thiomethyl-)-2,3,5,6-tetrabromo benzene, with the tris-(monooxethylate) of 1,3,5-tris-(thiomethyl-)-2,4,6-trichloro benzene, with tetra-(monooxethylates) of 1,4-dichloro-2,3,5,6-tetra-(thiomethyl-)benzene and 1,4-dibromo-2,3,5,6-tetra-(thiomethyl-)benzene, with the penta-(monooxethylate) of penta-(thiomethyl-)toluene and also with the hexa-(monooxethylate) of hexa-(thiomethyl)-benzene (= hexakis-(2-hydroxyethylthiomethyl-)benzene) and with the bis-(monooxethylate) of dipentene dimercaptan ($\hat{=}$ 2,9-para-methane dithiol).

6. A process for the preparation of the esters according to one of claims 1 to 5,
characterized in that
the 3-tert.butyl- and 3-tert.butyl-5-alkyl-4-hydroxyphenyl-(alkane)carboxylic acid and their derivatives are esterified with the oxethylates of the polythiol aromatics or dithiol cycloalkanes or re-esterified in the presence of known alkaline or neutral catalysts.

7. Use of the esters according to one of claims 1 to 5 for the stabilization of polymers.

8. Use of the esters according to one of claims 1 to 5 for the stabilization of polyolefins.

9. Use of the esters according to one of claims 1 to 5 for the stabilization of polyethylene and polypropylene.

10. Use of the esters according to one of claims 1 to 5 in a concentration of 0.02 to 3 per cent by weight, preferably 0.05 to 2 per cent by weight, referred to the polymer, for the stabilization of said polymers.

## Revendications

1. Esters d'acides 3-tert.Butyl ou 3-tert.Butyl-5-alcoyl-4-hydroxyphényl-(alcane-)carboxyliques de formule (I) :

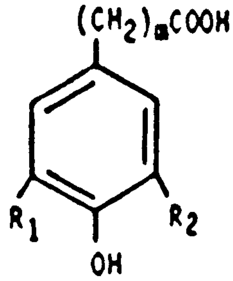

avec

$R_1$ = $-C(CH_3)_3$

$R_2$ = H, alcoyle en $C_1$ à $C_4$, de préférence $C(CH_3)_3$

m = 0 à 4, de préférence 2

avec des oxoéthylates de polythiolaromates de formule générale II et III, de préférence de formule II avec 2 à 6 groupes thiol :

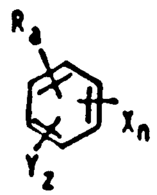

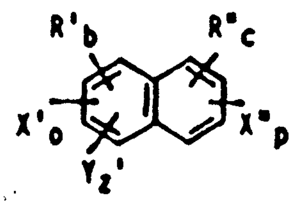

(II) (III)

ainsi qu'avec des oxoéthylates de dithiol cycloalcanes de formule générale IV qui renferment au maximum 6 unités d'oxyde d'alcoylène :

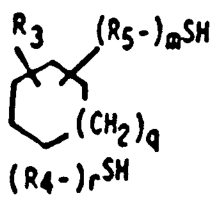

(IV)

avec $X = (CH_2)_m SH$
$X' = (CH_2)_r SH$
$X'' = (CH_2)_s SH$
} pour lequel ou m, r, s = 0 ou 1 peuvent être

R, R', R" = H, alcoyle, halogène, hydroxyle

$R_3$ = H, alcoyle en $C_1$ à $C_4$

$R_4, R_5$ = alcoylène en $C_2$ à $C_4$
q = 0 à 7 de préférence 1

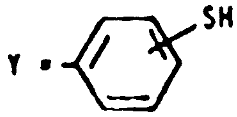

n = 2 à 6 pour z ou z' = 0
n = 1 à 5 pour z ou z' ≠ 0
0 + p = n
pour lequel
0 ≦ a, z ≦ a + z ≦ 6-n dans la formule II
0 ≦, b,c, z' ≦ 4 ≦ 8-n dans la formule III
peuvent être et la présence d'un groupe OH unique dans la formule II est exclue.

2. Ester de l'acide 3-(3,5-diter.Butyl -4-hydroxyphényl-)propionique de formule Ia :

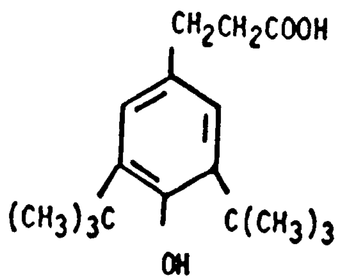

avec des oxoéthylates de polythiolaromates de formules générales II et III avec 2 à 6 groupes thiol ou avec des oxoéthylates de dithiol cycloalcanes de formule générale IV.

3. Ester selon la revendication 1 ou 2 avec des oxoéthylates de polythiolaromates de formule II avec 2 à 4 groupes thiol.

4. Ester selon la revendication 1 et 2 avec des oxoéthylates de polythiolaromates des formules II et III qui renferment de préférence une unité d'oxyde d'éthylène par groupe thiol de polythiolaromates comme le groupe 2-hydroxyéthythio.

5. Ester selon la revendication 1 et 2 avec des bis - (monooxyéthylates) de 1,4 - ou 1,2-bis-(thiométhyl)-benzène et de 1,4-bis-(thiométhyl-)-2,3,5,6-tétrabromobenzène avec le tris-(monooxoéthylate) du 1,3,5-Tris-(thiométhyl-)-2,4,6-trichlorobenzène, avec le tétra-(monooxoéthylate) du 1,4-dichloro-2,3,5,6-tétra-(thiométhyl-)benzène et du 1,4-dibromo-2,3,5,6-tétra-(thiométhyl-)benzène, avec le penta - (monooxoéthylate) du penta-(thiométhyl)toluène, et avec l'hexa-(monoéthylate) de l'hexa-(thiométhyl-)benzène (= Hexakis-(2-hydroxyéthylthiométhyl-benzène) ainsi qu'avec le bis-(monooxoéthylate) du dipentènedimercaptan ($\widehat{=}$ 2,9 - para-Menthanedithiol).

6. Procédé d'obtention des esters selon l'une des revendications 1 à 5, caractérisé en ce que l'on estérifie les acides 3-tert.Butyl- ou 3-tert.Butyl-5-alcoyl-4-hydrophényl-(alcane) carboxylique ou leurs dérivés avec les oxoéthylates des polythiolaromates ou des dithiolcycloalcanes ou transestérifie en présence de catalyseurs alcalins ou neutres connus en soi.

7. Utilisation des esters selon l'une des revendications 1 à 5 en vue de la stabilisation des polymères.

8. Utilisation des esters selon l'une des revendications 1 à 5 en vue de la stabilisation des polyoléfines.

9. Utilisation des esters selon l'une des revendications 1 à 5 en vue de la stabilisation du polyéthylène et

du polypropylène.

**10.** Utilisation des esters selon l'une des revendications 1 à 5 à une concentration de 0,02 à 3 % en poids, de préférence de 0,05 à 2 % en poids rapporté au polymère, en vue de la stabilisation de ces polymères.